# EUROPEAN PATENT APPLICATION

(11) **EP 4 471 789 A1**
(43) Date of publication of application: **04.12.2024**
(21) Application number: 24179147.4
(22) Date of filing: 30.05.2024
(51) Int. Cl.: G16C 20/30, G16C 20/70

(54) **ADVANCED METHODS AND SYSTEMS FOR DETERMINING PROPERTIES OF A MOLECULE WITH MACHINE LEARNING**

(30) Priority: 30.05.2023 US 202318325787
(71) Applicant: Dassault Systèmes Americas Corp., Waltham, MA 02451 (US)
(72) Inventor: Schwöbel, Johannes Alfred Hugo, 40764 Langenfeld (DE); Schweizer, Sabine, 74918 Angelbachtal (DE); Skinner, Kwan, Mesa, AZ 85212 (US); Subramanian, Lalitha, Newtown, PA 18940 (US)
(74) Representative: Bandpay & Greuter

(57) **Abstract**

Embodiments determine properties of a molecule in an environment. One such embodiment constructs one or more three-dimensional (3D) structure models that indicate positions of atoms of the molecule. For each of the constructed one or more 3D structure models: (i) a surface model is generated that represents the environment, where the surface model includes a plurality of segments and the generated surface model defines a relationship between the indicated positions of the atoms of the 3D structure model and the plurality of segments and (ii) using a machine learning model, charge (e.g., electric charge) and chemical potential of each segment of the plurality of segments are predicted based on the 3D structure model and the generated surface model. An embodiment further predicts, using a supplemental machine learning model, energy corresponding to the 3D structure model based on the 3D structure model and the generated surface model.

## Description

### BACKGROUND

Existing approaches for determining quantum-chemical properties of a molecule in a condensed environment are limited to small or medium-sized molecules. Further, existing approaches of continuum solvation models are computationally demanding and/or less accurate, depending on size and complexity of the molecule.

### SUMMARY

Therefore, functionality with improved accuracy and computational efficiency for determining properties of molecules, e.g., large molecules, in condensed environments is needed. Embodiments provide such functionality.

One such embodiment provides this functionality by constructing one or more three-dimensional (3D) structure models that indicate positions of atoms of the molecule. In turn, such an embodiment, for each of the constructed one or more 3D structure models: (i) generates a surface model representing the environment, where the surface model includes a plurality of segments and the generated surface model defines a relationship between the indicated positions of the atoms of the 3D structure model and the plurality of segments and (ii) predicts, using a machine learning model, charge (e.g., electric charge) and chemical potential of each segment of the plurality of segments based on the 3D structure model and the generated surface model.

In addition, certain embodiments provide functionality to vet candidate molecules for their properties in an environment. An example embodiment for vetting candidate molecules is directed to a method that receives one or more user requirements, determines the properties of each candidate molecule of a plurality of candidate molecules, and selects a given molecule, from among the plurality of candidate molecules, based on the determined properties of the given molecule and the received one or more user requirements.

Certain embodiments relate to fluid phase thermodynamics and simulation. For example, some embodiments are applicable to, e.g., organic and organo-metallic molecules in general. The size of molecular systems to which certain embodiments of the described approaches can be applied is not limited to small or medium sized molecules. Advantageously, certain embodiments can also be used for large molecular systems like polymers or biomolecules, among other examples. Further, some embodiments can be used to predict and calculate thermodynamic equilibrium properties of molecular systems in liquid and vapor/gas phases. The determination of such properties is important across many different industries, e.g., material science, pharmaceutical, life science, medical, consumer packaged goods, cosmetics, polymers, and coating, etc. In particular, some embodiments can be applied for excipient screening for, e.g., chemical engineering, drug development, formulation design of personal and consumer care products, design of packaging material, and plastics recycling, among other examples.

Further, certain embodiments can be used for calculating thermodynamic equilibrium properties of, e.g., large organic, molecules and ions in the liquid phase in a fast way with reliable accuracy. Thermodynamic equilibrium properties may include, for example, activity coefficients, vapor pressures, solubilities, free energy of solvation, partition coefficients, reactivities, and other related properties known in the art. Through some embodiments, large molecules can also be modeled and analyzed, which is important for industrial applications such as the development of novel compounds and materials, e.g., biologics in the pharmaceutical industry or biodegradable polymers for polymer manufacturers, among other examples.

Certain embodiments can construct and calculate surface charge densities and corresponding potentials of organic and organo-metallic molecules and ions in a dielectric continuum using an innovative workflow involving machine learning. The method according to an embodiment is computationally efficient and capable of modeling and analyzing large molecules, including, for example, polymers. Some embodiments can extend the applicable domain of methods that use the charge density to predict thermodynamics properties to, e.g., complex polymeric or biochemical systems.

Moreover, certain embodiments can calculate thermodynamic equilibrium properties involving the liquid phase of conventional and novel large organic molecules and ions in a fast way with reliable accuracy. The efficient method according to embodiments reduces the computational time by several orders of magnitude, thus making it possible to run high-throughput screenings and to extend the applicable domain of thermodynamic equilibrium predictions to macromolecules, e.g., polymers or biomolecules. Some embodiments provide a computational method for constructing and calculating segment-wise surface charge densities and potentials of molecules and ions in a dielectric continuum using a workflow involving machine learning without prior knowledge apart from atom types (e.g., represented by element symbols or atomic numbers) and atom 3D coordinates, while taking a segment-specific local 3D chemical environment into account.

One such embodiment delivers this functionality via a workflow including: (i) providing a molecular geometry (conformer) or a set of molecular geometries (conformer sets) as input; (ii) constructing conformer-specific segments on a solvent-accessible surface area; (iii) approximating segment-specific information in an efficient process involving a trained machine learning model, plus predicting molecular energies; and (iv) writing out collected information for segments and energies for each conformer of consideration. According to an embodiment, the aforementioned information is written in a so-called COSMO file or other suitable file format known to those in the art. Finally, the COSMO (or other format) file information can be processed by statistical thermodynamics software packages, e.g., BIOVIA^{®} COSMOtherm^{®}, or other suitable software packages known in the art, to predict thermodynamic equilibrium properties for molecules in condensed environments.

An example embodiment is directed to a computer-implemented method for determining properties of a molecule in an environment, e.g., a condensed-phase environment, such as a liquid, solvent, or excipient. The method begins by constructing one or more 3D structure models that indicate positions of atoms of the molecule. Next, for each of the constructed one or more 3D structure models, the method: (i) generates a surface model representing the environment, where the surface model includes a plurality of segments and the generated surface model defines a relationship between the indicated positions of the atoms of the 3D structure model and the plurality of segments and (ii) predicts, using a machine learning model, charge and chemical potential of each segment of the plurality of segments based on the 3D structure model and the generated surface model. In an embodiment, the relationship between the indicated positions of the atoms of the 3D structure model and the plurality of segments may be defined by the generated surface model using a relationship between (i) center positions or coordinates of the constructed segments on a solvent-accessible surface and (ii) the indicated positions of the atoms of the 3D structure model. According to an embodiment, the solvent-accessible surface is defined as a boundary of all positions in space, which can be taken by a center of a solvent or probe sphere. According to an aspect, the generated surface model stores separate coordinates of each atom position and each segment position. In such an implementation, these stored coordinates define relationships between positions of atoms and the segments. Further, it is noted that the surface model including the plurality of segments may be generated by any suitable cavity construction method known to those of skill in the art, including, for example, known cavity construction methods described or discussed herein.

Certain embodiments may predict charge and chemical potential using a respective machine learning model for each property. For example, in an aspect, the machine learning model includes a first machine learning model and a second machine learning model. According to one such aspect, predicting the charge and the chemical potential of each segment of the plurality of segments based on the 3D structure model and the generated surface model includes predicting, using the first machine learning model, electric charge of each segment of the plurality of segments based on the 3D structure model and the generated surface model, and predicting, using the second machine learning model, the chemical potential of each segment of the plurality of segments based on the 3D structure model and the generated surface model.

According to another embodiment, determining the properties of the molecule in the environment further includes, for each constructed 3D structure model, predicting, using a supplemental machine learning model, energy corresponding to the 3D structure model based on the 3D structure model and the generated surface model.

According to another example embodiment, the machine learning model includes a neural network. In an aspect, the neural network includes one or more hidden layers and the neural network is configured to employ an activation function at one or more nodes of the one or more hidden layers. According to an implementation, the activation function is one of a rectified linear unit (ReLU) activation function and a softmax function. However, embodiments are not limited to the particular activation functions listed above and, instead, any suitable activation function known in the art may be employed.

In yet another example embodiment, the method further includes training the machine learning model based on a training data set. According to an aspect, the machine learning model includes a neural network, and training the machine learning model based on the training data set includes training the neural network by iteratively updating one or more network weights of the neural network based on the training data set. In an implementation, iteratively updating the one or more network weights of the neural network based on the training data set includes employing one or more of an adaptive moment estimation (Adam) solver algorithm and an early stopping algorithm. According to an example embodiment, the training data set includes data for one or more of example molecules, example conformers, example segments, example segment charges, example segment chemical potentials, and example continuum model energies.

In an embodiment, predicting, using the machine learning model, the charge and the chemical potential of each segment of the plurality of segments based on the 3D structure model and the generated surface model includes deriving input feature data based on the 3D structure model. Further, such an embodiment predicts, using the machine learning model, the charge and the chemical potential of each segment of the plurality of segments based on the 3D structure model, the generated surface model, and the derived input feature data. According to one such embodiment, the derived input feature data includes an indication of one or more of atom type, atom-atom distance, atom-segment distance, bond type, bond angle, torsion angle, formal charge, 3D atom position, and atom-type specific features.

An example embodiment further includes receiving one or more user requirements. Such an embodiment then evaluates candidate molecules in relation to the received user requirements. For each candidate molecule of a plurality of candidate molecules, an example embodiment performs the constructing and the determining the properties and selects a given molecule from among the plurality of candidate molecules based on the determined properties of the given molecule and the received one or more user requirements. According to an aspect, the one or more user requirements may include, e.g., molecular or ionic input structures, input notations, and/or connectivity tables.

In another example embodiment, predicting, using the machine learning model, the charge and the chemical potential of each segment of the plurality of segments based on the 3D structure model and the generated surface model includes correcting one or more residual charges of the plurality of segments and determining an overall formal charge of the plurality of segments based on the corrected one or more residual charges of the plurality of segments. According to one such example embodiment, the determined overall formal charge is the predicted charge of the plurality of segments.

In an example embodiment, each 3D structure model of the constructed one or more 3D structure models corresponds to a respective conformer of the molecule.

Various types of information and/or models may be used in constructing the one or more 3D structure models. For instance, according to yet another example embodiment, the one or more 3D structure models indicating the positions of the atoms of the molecule are constructed based on indications of one or more of atom type, coordinates, and chemical connectivity, among other examples. In another aspect, the one or more 3D structure models can be constructed by employing one or more of: rule-based geometrical models, force fields, and quantum-chemically derived geometrical models, among other nonlimiting examples. According to an embodiment, quantum-chemically derived geometrical models may include, e.g., a tight-binding model, a semi-empirical model, a density functional theory derived geometrical model, or any combinations thereof.

In another implementation, the surface model representing the environment is generated using a cavity construction model. Some embodiments may employ any suitable cavity construction model known in the art, such as a COSMO FINE Cavity construction model.

Another example embodiment is directed to a computer-based system for determining properties of a molecule in an environment. The system includes a processor and a memory with computer code instructions stored thereon. In such an embodiment, the processor and the memory, with the computer code instructions, are configured to cause the system to implement any embodiments or combination of embodiments described herein.

Yet another example embodiment is directed to a cloud computing implementation for determining properties of a molecule in an environment. Such an embodiment is directed to a computer program product executed by a server in communication across a network with one or more clients, where the computer program product comprises instructions which, when executed by one or more processors, cause the one or more processors to implement any embodiments or combination of embodiments described herein.

It is noted that embodiments of the method, system, and computer program product may be configured to implement any embodiments, or combination of embodiments, described herein.

### BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.
FIG. 1 is a flowchart of a method for determining properties (e.g., continuum solvation model properties) of a molecule in an environment according to an embodiment.
FIG. 2 is a simplified block diagram of a system for determining properties (e.g., continuum solvation model properties) of molecules in environments according to an embodiment.
FIG. 3 illustrates an exemplary workflow for determining properties of a molecule in an environment according to an embodiment.
FIG. 4 is a simplified block diagram of a computer system for determining properties of a molecule in an environment according to an embodiment.
FIG. 5 is a simplified block diagram of a computer network environment in which embodiments of the present invention may be implemented.

### DETAILED DESCRIPTION

A description of example embodiments follows.

Chemistry, i.e., chemical interactions/reactions, takes place mostly in the liquid or solvent phase. Theoretical models to embed molecules in a solvent continuum are known. For instance, existing continuum solvation models approximate a solvent as a dielectric continuum surrounding solute molecules outside of a molecular cavity. The cavity surface is approximated by segments, e.g., hexagons, pentagons, or triangles. Models that approximate a solvent as a dielectric continuum may be referred to as dielectric continuum solvation models (DCSMs), and include the widely used Polarizable Continuum Model (PCM). Another example conventional DCSM is the Conductor-like Screening Model (COSMO), which derives polarization charges of the continuum, caused by polarity of the solute, from a scaled-conductor approximation [1] (bracketed numbers in this document refer to the enumerated list of references hereinbelow). COSMO is one of the most applied calculation methods for determining electrostatic interactions of a molecule with a solvent or liquid environment. Output data from COSMO or other known DCSM approaches (generally referred to herein as "COSMO information" or "COSMO-type information") may include, for example, segment-wise surface charge densities and chemical potentials. In general, DCSMs each approximate solvent influence in a similar way by approximating a dielectric continuum.

COSMO information may in turn be used to calculate chemical potential of a molecule in a solvent or solvent mixture. For example, this may be performed via the existing COSMO-RS (where "RS" stands for "real solvents") approach, which takes as input previously calculated and stored COSMO information. The COSMO-RS approach involves modeling a set of physicochemical interaction terms between the molecule and its liquid environment as a function of pairwise charge densities of particular segments of a molecular cavity [2].

Chemical potential data produced by, e.g., COSMO-RS, may then form the basis for calculating general thermodynamic equilibrium properties. Such thermodynamic properties may include, for example, activity coefficients, solubility, partition coefficients, vapor pressure, and free energy of solvation. The COSMO-RS method was developed to provide a general prediction method with no need for system-specific adjustment. The method is widely applied by academia and the chemical and pharmaceutical industries.

Currently used approaches to generate COSMO information rely on either quantum-chemical calculations [1,2], partially complemented by machine learning post corrections [3], or fragmentation approaches [4].

Quantum-chemical calculations have, for example, the drawback that they can be computationally very demanding, especially for larger molecular systems. Typically, quantum-chemical calculations are performed on high-performance computing (HPC) clusters for relevant conformer sets. The routine application of quantum-chemical methods is thus limited to settings with trained users who have access to HPC clusters. Such computing-intensive calculations limit quantum-chemical methods to small molecules, with molecular weights significantly below 1 kilodalton (kDa). In contrast, many industrially-relevant chemicals and biochemicals, such as polymers, surfactants, proteins, or biologicals, etc., are in the range of 10 to 100 kDa or even more. Modern drug molecules also reach this molecular weight limitation very quickly.

Although some existing quantum-chemical methods, for example semi-empirical methods, have a reduced computational demand, employing such methods leads to insufficient polarity distributions, which in turn compromises the subsequent calculation of thermodynamic properties.

Fragmentation approaches do not properly reflect the 3D conformational space and its impact on charge distribution. For example, in certain 3D configurations, the conformational space's impact on charge distribution is affected by the formation of intramolecular hydrogen bonds.

Some embodiments described herein provide, as one example, the advantage of reducing the computational effort for calculating surface charge densities from multiple days or even weeks to a few seconds or even less time, with accuracy comparable to that of quantum-chemical calculations. In certain embodiments, quantum-chemical calculations are replaced by an efficient workflow involving a set of machine learning procedures. In addition, numerical artifacts arising from derivations of quantum-chemical equations can be avoided or smoothed out by the machine learning procedures implemented by some embodiments. For this reason, certain embodiments provide thermodynamic property calculations with increased prediction accuracy compared to existing approaches. The fast machine learning procedures of some embodiments take a molecular geometry or a set of molecular geometries as input. Certain embodiments efficiently predict charges, e.g., screening charges, and chemical potentials for molecular surface segments, by reflecting a local chemical environment of a segment in a particular molecule or conformer. In an embodiment, segments are constructed in a 3D space, by employing any suitable cavity construction model known in the art, such as a COSMO FINE Cavity construction model as described in [5] (which is herein incorporated by reference in its entirety), or, for example, any other existing cavity construction methods reviewed by [5] (which are herein incorporated by reference in their entirety).

### Example Method Embodiments

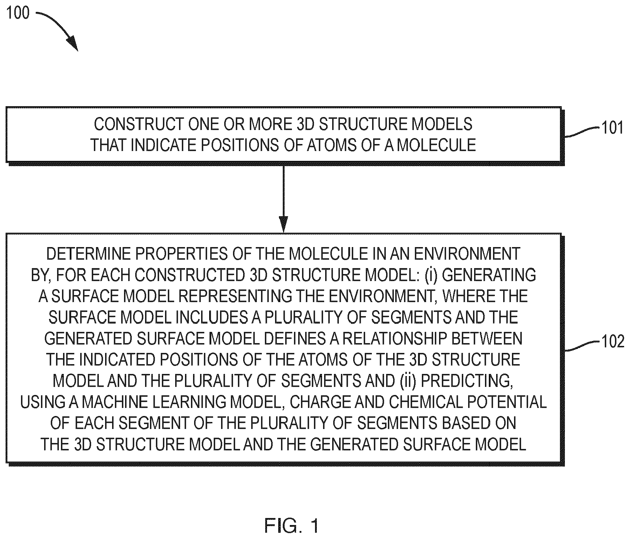

FIG. 1 illustrates one such example method embodiment 100. The method 100 is a computer-implemented method for determining properties of a molecule in an environment. The method 100 begins at step 101 by constructing, e.g., in computer memory, one or more 3D structure models that indicate positions of atoms of the molecule. In an embodiment, constructing the one or more 3D structure models at step 101 may be performed using any suitable technique known to those of skill in the art. For example, embodiments may construct 3D structure models using known software platforms, e.g., BIOVIA^{®} platforms such as Pipeline Pilot^{®}, COSMOquick^{®}, Materials Studio^{®}, Discovery Studio^{®}, or COSMOconf^{®}, or other platforms such as TURBOMOLE. According to an embodiment, each of the constructed one or more 3D structure models corresponds to a respective conformer of the molecule. Various types of information and/or models may be used in constructing the one or more 3D structure models at step 101. For instance, in an embodiment of the method 100, the one or more 3D structure models are constructed (101) based on indications of one or more of atom type, coordinates, and chemical connectivity. Chemical connectivity refers to how atoms are connected spatially to each other. Further, in some embodiments, the models may be constructed based on measurements/observations of real-world molecules and the resulting models reflect the real-world measured/observed properties. According to another example embodiment of the method 100, constructing the one or more 3D structure models (101) includes employing one or more of: rule-based geometrical models, force fields, and quantum-chemically derived geometrical models, among other nonlimiting examples. In an embodiment, quantum-chemically derived geometrical models may include, e.g., a tight-binding model, a semi-empirical model, a density functional theory derived geometrical model, or any combinations thereof.

To continue, the method 100 determines the properties of the molecule in the environment at step 102. The properties of the molecule are determined at step 102 by, for each of the constructed one or more 3D structure models (from step 101): (i) generating, e.g., in computer memory, a surface model representing the environment, where the surface model includes a plurality of segments and the generated surface model defines a relationship between the indicated positions of the atoms of the 3D structure model and the plurality of segments and (ii) predicting, using a machine learning model, charge (e.g., electric charge) and chemical potential of each segment of the plurality of segments based on the 3D structure model and the generated surface model. In an embodiment, generating the surface model at step 102 may be performed using any suitable technique known to those of skill in the art.

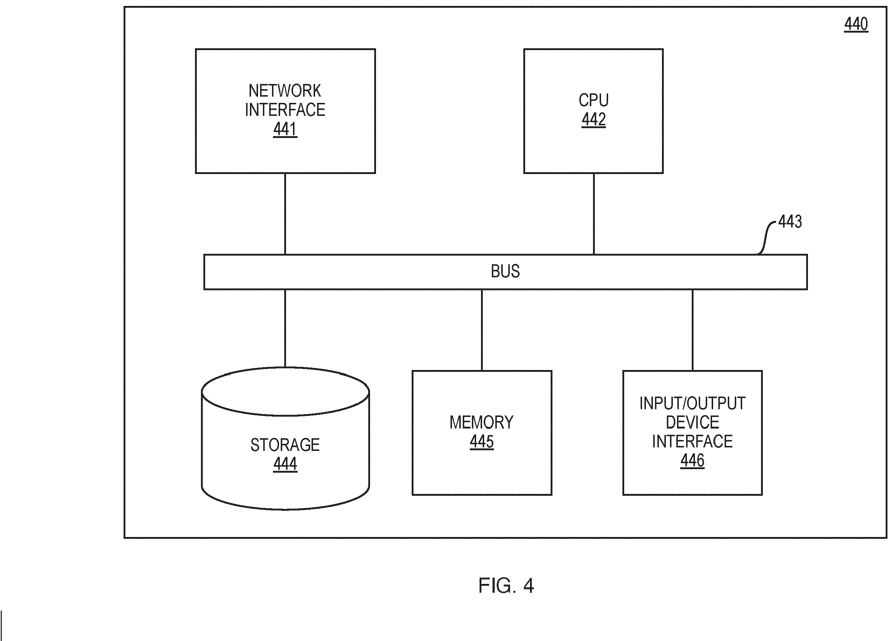

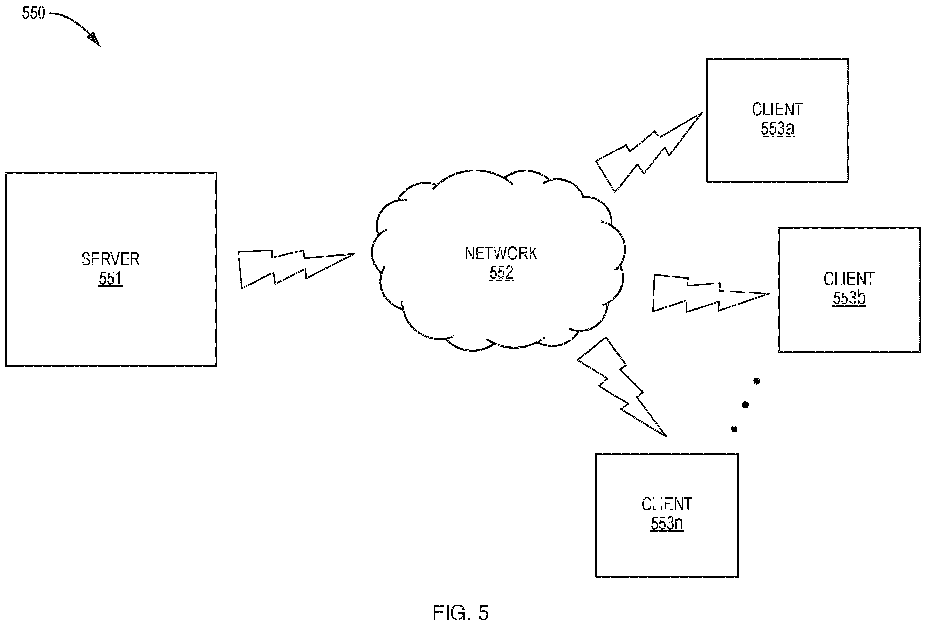

As noted, the method 100 is computer implemented and, as such, the functionality and effective operations, e.g., the constructing (101) and determining (102) are automatically implemented by one or more digital processors. Moreover, the method 100 can be implemented using any computer device or combination of computing devices known in the art. Among other examples, the method 100 can be implemented using the computer system 440 described hereinbelow in relation to FIG. 4 and the computer network environment 550 described hereinbelow in relation to FIG. 5.

At step 102, the method 100 may use a first machine learning model to predict electric charge and a second machine learning model to predict chemical potential. An embodiment of the method 100 predicts the charge of each segment of the plurality of segments based on the 3D structure model and the generated surface model at step 102 using the first machine learning model. In such an embodiment, the 3D structure model (and/or characteristics thereof) and the generated surface model (and/or characteristics thereof) are provided as inputs to the first machine learning model and the first machine learning is configured to output electric charges of each segment responsive to the input. Similarly, an embodiment predicts, at step 102 using the second machine learning model, the chemical potential of each segment of the plurality of segments based on the 3D structure model and the generated surface model. Such an embodiment inputs the 3D structure model (and/or characteristics thereof) and the generated surface model (and/or characteristics thereof) to the second machine learning model and the second machine learning is configured to output chemical potentials of each segment responsive to the input.

In an embodiment of the method 100, determining the properties of the molecule in the environment at step 102 further includes predicting, using a supplemental machine learning model, energy corresponding to the 3D structure model based on the 3D structure model and the generated surface model. Such an embodiment inputs the 3D structure model (and/or characteristics thereof) and the generated surface model (and/or characteristics thereof) to the supplemental machine learning model and the supplemental machine learning model is configured to output energy corresponding to the 3D structure model responsive to the input.

In an embodiment of the method 100, predicting, using the machine learning model, the charge (e.g., electric charge) and the chemical potential of each segment of the plurality of segments based on the 3D structure model and the generated surface model (102) includes deriving input feature data based on the 3D structure model. Such an embodiment predicts, using the machine learning model, the charge and the chemical potential of each segment of the plurality of segments based on the 3D structure model, the generated surface model, and the derived input feature data. According to an embodiment, the derived input feature data includes an indication of one or more of atom type, atom-atom distance, atom-segment distance, bond type, bond angle, torsion angle, formal charge, 3D atom position, and atom-type specific features.

In an embodiment of the method 100, predicting, using the machine learning model, the charge (e.g., electric charge) and the chemical potential of each segment of the plurality of segments based on the 3D structure model and the generated surface model (102) includes correcting one or more residual charges of the plurality of segments and determining an overall formal charge of the plurality of segments based on the corrected one or more residual charges of the plurality of segments. According to one such example embodiment, the determined overall formal charge is the predicted charge of the plurality of segments.

In an embodiment of the method 100, generating the surface model representing the environment (102) includes employing a cavity construction model. An embodiment may employ any suitable cavity construction model known in the art, such as a COSMO FINE Cavity construction model as described in [5], or, for example, any other existing cavity construction methods reviewed by [5]. In an embodiment of the method 100, the machine learning model used at step 102 includes a neural network. Further, in yet another embodiment, the neural network includes one or more hidden layers and the neural network is configured to employ an activation function at one or more nodes of the one or more hidden layers. According to an embodiment, the activation function is one of a rectified linear unit (ReLU) activation function and a softmax function. However, embodiments are not limited to the particular activation functions listed above; instead, any suitable activation function known in the art may be employed. Although neural networks are discussed herein, those of skill in the art will recognize that embodiments of the method 100 are not limited to such techniques. Rather, embodiments of the method 100 may utilize any suitable known machine learning or statistical learning methods at step 102 to determine the charge and chemical potential.

According to an embodiment, the method 100 further includes training the machine learning model based on a training data set. In an embodiment, the machine learning model includes a neural network, and training the machine learning model based on the training data set includes training the neural network by iteratively updating one or more network weights of the neural network based on the training data set. Further, in yet another embodiment, iteratively updating the one or more network weights of the neural network based on the training data set includes employing one or more of an adaptive moment estimation (Adam) solver algorithm and an early stopping algorithm. It is noted that embodiments are not limited to the particular algorithms listed above; instead, any suitable algorithm(s) known in the art may be employed. According to an example embodiment, the training data set includes data for one or more of: example molecules, example conformers, example segments, example segment charges, example segment chemical potentials, and example continuum model energies. In yet another embodiment, the training data set may include a voluminous COSMO file collection that covers an organic-chemical space spanning, for example, solvents, industrial chemicals, pharmaceuticals, ionic protonation states, ions, and ionic liquids (cations and anions, also including multiply charged ions).

According to an embodiment, the method 100 further includes receiving one or more user requirements. In one such example embodiment, for each candidate molecule of a plurality of candidate molecules, the method 100 then performs the constructing (101) and the determining the properties (102) and selects a given molecule from among the plurality of candidate molecules based on the determined properties of the given molecule and the received one or more user requirements. According to an aspect, the one or more user requirements may include, e.g., molecular or ionic input structures, input notations, and/or connectivity tables.

Further, in another embodiment of the method 100, properties of candidate molecules are determined from real-world measurements of said candidate molecules. These measured properties are then used at step 101 to construct computer-based models of the candidate molecules with said properties. In turn, these models (which reflect the real-world measured/observed properties of the candidate molecules) are used at step 102 to determine the charge and chemical potential of each candidate molecule. A given candidate molecule that meets desired criteria can then be selected for use in a real-world application, such as formulating a research plan, optimizing formulations to improve product properties in a desired way, designing sustainable polymers for recycling and other processes involving polymers, identifying a suitable pharmaceutical excipient in the context of drug development, focusing a set of experiments, and reducing material waste by improving the efficiency of experiments, among other examples.

Certain embodiments of the method 100 may use file formats/structures of various known software tools, such as COSMO, to store to various properties determined/predicted by the method 100, e.g., charge (such as electric charge), chemical potential, energy, and any other data/values described herein. Some embodiments may also generate and store data in the cloud, e.g., the 3DEXPERIENCE^{®} platform. However, embodiments are not limited to a particular file format/structure or cloud platform; instead, any suitable file format/structure or cloud platform known in the art may be used.

### Example System Embodiment

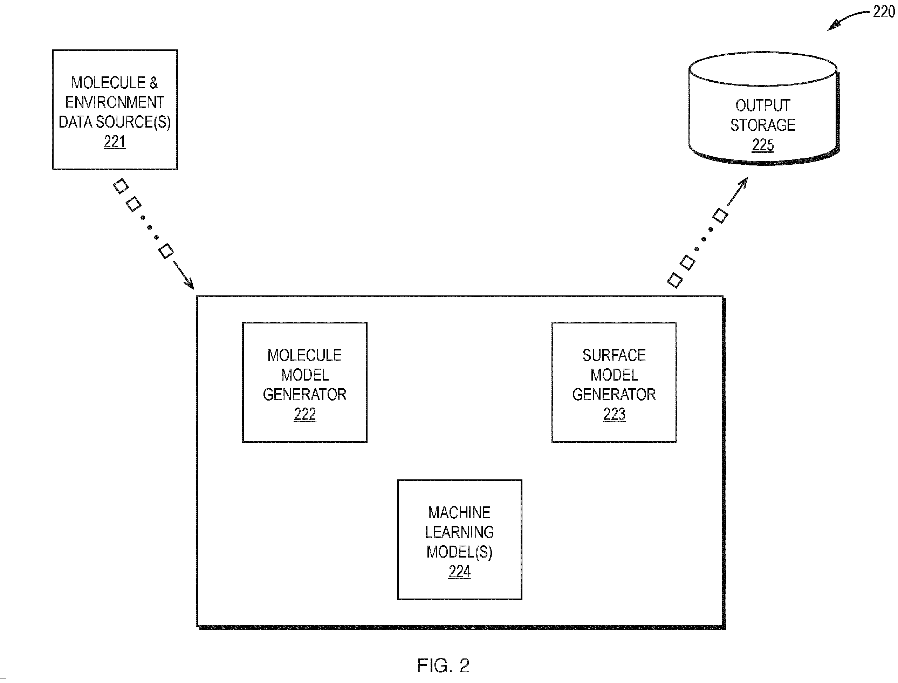

FIG. 2 is a simplified block diagram of a system 220 for determining properties of molecules in environments according to an embodiment.

As shown in FIG. 2, in an aspect, system 220 includes one or more data sources 221, molecule model generator 222, surface model generator 223, one or more machine learning models 224, and output storage 225.

In an embodiment, data source(s) 221 may include molecule data, i.e., data about a molecule for which properties are to be determined, as well as environment data, i.e., data about the molecule's environment. According to an implementation, data source(s) 221 may be provided by a user of the system 220. In an example embodiment, data source(s) 221 may be used as input to, e.g., molecule model generator 222 and surface model generator 223.

According to an embodiment of the system 220, using data about a molecule provided by data source(s) 221, molecule model generator 222 may construct one or more 3D structure models that indicate positions of atoms of the molecule.

Likewise, in an aspect, using data about an environment provided by data source(s) 221, surface model generator 223 may, for each 3D structure model of interest constructed by the molecule model generator 222, generate a surface model representing the environment. In such an embodiment, each surface model includes a plurality of segments and each generated surface model defines a relationship between indicated positions of the atoms of the 3D structure model and the plurality of segments.

According to an implementation, based on each 3D structure model of the one or more 3D structure models constructed by the model generator 222 and a respective surface model generated by surface model generator 223, machine learning model(s) 224 may predict, e.g., charge (such as electric charge) and chemical potential, of each segment of a plurality of segments of the surface model. In an embodiment, machine learning model(s) 224 may include a first machine learning model and a second machine learning model, where the first machine learning model predicts electric charge, and the second machine learning model predicts chemical potential. Further, according to an aspect, machine learning model(s) 224 may include a supplemental machine learning model that predicts energy corresponding to the 3D structure model based on the 3D structure model and the generated surface model. In an embodiment, machine learning model(s) 224 may include a neural network.

In an example embodiment, properties, e.g., charge, chemical potential, and/or energy, predicted by machine learning model(s) 224 may be output to storage 225. Further, according to an embodiment, once recorded in storage 225, such output data may then be used for additional processing, such as calculating thermodynamic properties, among other examples.

It should be noted that system 220 can implement any embodiments described herein, e.g., method 100 described hereinabove with respect to FIG. 1, to determine properties of molecules in environments.

### Exemplary Workflow

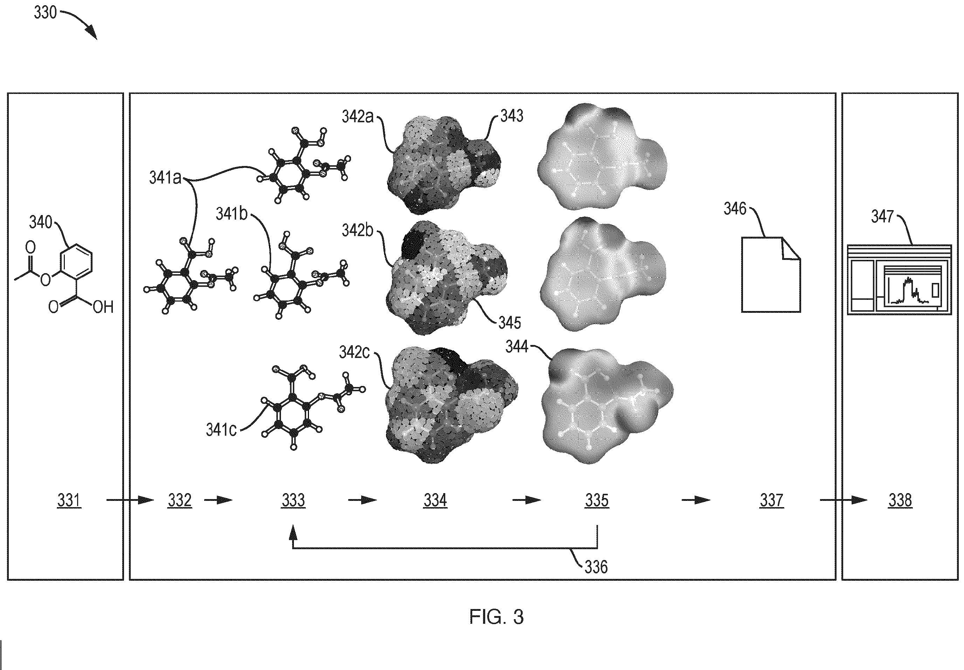

FIG. 3 illustrates an exemplary workflow 330 for determining properties of a molecule in an environment according to an embodiment. Workflow 330 can implement any embodiments described herein, e.g., method 100 and system 220 described hereinabove in relation to FIGs. 1 and 2, respectively, to determine properties of molecules in environments.

In an embodiment, at step 331 of workflow 330, an input structure is obtained, e.g., responsive to a user action. According to an example embodiment, the input structure may be a structure of a molecule of which properties in an environment are to be determined. FIG. 3 illustrates an example two-dimensional (2D) molecule structure 340 that may be obtained at step 331 of the workflow 330.

Next, according to an embodiment, at step 332, 3D structure generation for the molecule may occur. FIG. 3 illustrates the constructed 3D structure 341a of the 2D molecule structure 340. In an implementation, optional conformer generation for the molecule may also take place at step 333. For example, FIG. 3 depicts the additional conformers 341b-c. According to an embodiment, the 3D structure generation of step 332, and the optional conformer generation of step 333, may include constructing, e.g., in computer memory, one or more 3D structure models (341a-c) indicating positions of atoms of the molecule. In an aspect, constructing the one or more 3D structure models (341a-c) may be performed using any suitable technique known to those of skill in the art. According to an example embodiment, each of the constructed one or more 3D structure models (341a-c) corresponds to a respective conformer of the molecule (340).

Continuing with respect to FIG. 3, according to an embodiment, the workflow 330 determines the properties of the molecule in the environment. In an aspect, the properties of the molecule are determined by, for each of the constructed one or more 3D structure models 341a-c (from step 332, and optionally step 333): (i) generating, e.g., in computer memory, a surface model (342a-c) representing the environment, where the surface model 342a-c includes a plurality of segments (e.g., tiles, which may be hexagon-shaped, such as tile 345 shown in FIG. 3) and the generated surface model 342a-c defines a relationship between the indicated positions of the atoms of the 3D structure model 342a-c and the plurality of segments and (ii) predicting, e.g., at step 335, using a machine learning model, charge and chemical potential of each segment of the plurality of segments based on the 3D structure model 342a-c and the generated surface model 342a-c. According to an example embodiment, generating the surface model may include performing cavity construction and tiling into segments at step 334. It should be noted that generating the surface model-including, for example, the cavity construction and tiling into segments of step 334-may be performed using any suitable technique known to those of skill in the art. According to another example embodiment, as shown by, e.g., region 343 of FIG. 3, when presented visually, individual segments may be shaded/colored to distinguish between segments mapped to or corresponding to a particular atom of the molecule.

According to an embodiment, step 335 of workflow 330 may include using one or more machine learning models to, variously, predict charge and chemical potential for each segment and energy for each conformer. For example, in an implementation, a first machine learning model may be used to predict electric charge and a second machine learning model may be used to predict chemical potential. According to an aspect, a supplemental machine learning model may be used to predict energy. In an embodiment, as shown by, e.g., region 344 of FIG. 3, when presented visually, individual segments may be shaded/colored to distinguish between segments with negative, positive, or neutral predicted charges.

To continue with FIG. 3, in an example embodiment, as a processing option, geometry optimization may be performed at step 336 of workflow 330 by varying the 3D structure model and its optimization towards an energy minimum, followed by repeating steps 333, 334, and 335. According to one such embodiment, this processing option may be iterated one or more times.

In yet another example embodiment, after properties, e.g., charge, chemical potential, and/or energy, are predicted by the one or more machine learning models, values of the properties may be written or saved to one or more output file(s) 346 at step 337 of workflow 330. Further, according to an embodiment, after being saved at step 337, such output data may then be used for additional processing at step 338, such as calculating thermodynamic properties and/or generating thermodynamic prediction(s) 347, among other examples.

### Molecular or Macromolecular Geometry

Certain embodiments may construct one or more 3D structure models relating to a particular molecule of interest, e.g., at step 101 of the method 100.

Some embodiments may take a molecular geometry (single conformer) or a set of molecular geometries (conformer set) as input. Conformers may include, for example, tautomeric and/or protonation states. Molecular geometries may be defined by individual atomic coordinates and atom types.

In addition, certain embodiments may perform any one or more of the following operations to construct models that indicate positions of atoms: (i) a conformational search to generate a respective set of 3D structure models of conformers, (ii) a tautomeric search to generate a respective set of 3D structure models of a single conformer or a set of conformers for each tautomer, (iii) a search for relevant protonation states to generate a respective set of 3D structure models of a single conformer or a set of conformers for each protonation state, (iv) creation of different substitution patterns, and (v) creation of different mutant or variant structures.

According to an embodiment, constructing a 3D structure model includes receiving one or more molecular geometries from any source known in the art, such as industry-standard fast 3D structure generators, X-ray structures, force-fields, tight-binding methods, or semi-empirical or density-functional calculations.

For example, in an embodiment, BIOVIA^{®} Pipeline Pilot^{®} or BIOVIA^{®} COSMOquick^{®} software may be utilized to generate molecular geometries and conformer sets from chemical connectivity information in molecule files stored as, e.g., SDF (structure data file), MOL (Molfile), PDB (Protein Data Bank), or related molecule file formats, or molecular input line notations, SMILES (simplified molecular-input line-entry system), among other examples. Molecule builders in BIOVIA^{®} Materials Studio^{®} or BIOVIA^{®} Discovery Studio^{®} that utilize force-fields to generate reliable 3D geometries for molecules and macromolecule systems, e.g., CHARMM (Chemistry at Harvard Macromolecular Mechanics) or COMPASS (Condensed-phase Optimized Molecular Potentials for Atomistic Simulation Studies) force fields, may also be utilized by some embodiments to construct 3D structure models. Additionally, BIOVIA^{®} COSMOconf^{®} and TURBOMOLE, for example, support complementary methods to generate 3D structures, e.g., RDKit related force-fields, MOP AC (Molecular Orbital PACkage) semi-empirical methods, or xTB semi-empirical extended tight-binding, that can be employed by certain embodiments. In some embodiments, density-functional calculations can be performed by quantum-chemical packages, e.g., TURBOMOLE, to generate 3D structures. It is noted that embodiments are not limited to the particular software platforms or file formats described herein; instead, any suitable software platforms or file formats known in the art may be used.

Molecular 3D geometries may be generated by, e.g., the ETKDG (Experimental-Torsion basic Knowledge Distance Geometry) method of the RDKit cheminformatics library. Other known sources and techniques for molecular geometries may be used in addition to those discussed herein.

It is also noted that in some embodiments, atomic coordinates and atom types are used to determine molecule properties, e.g., continuum solvation model properties. Unlike existing approaches, such embodiments do not explicitly rely on, e.g., bond information, connectivity information, functional groups, fingerprints, segment-specific information, molecular surface-specific information, energies, or potentials.

### Construction of Solvent-Accessible Surface Segments

Certain embodiments may generate, e.g., at step 102 of the method 100, a surface model representing an environment, such as a continuum solvent environment. Generated surface models may include a plurality of segments. Further, generated surface models may define a relationship between indicated positions of atoms of a 3D structure model and the plurality of segments. According to an embodiment, a surface model may encapsulate a conformer (represented by a 3D structure model) in a tiled cavity where the tiled cavity represents a continuum solvent environment. In some embodiments, the tiled cavity representing the continuum solvent environment may be calculated by any suitable technique known in the art, such as a COSMO solvation model. According to an implementation, a plurality of segments of a given surface model may represent a conformer-specific solvent-accessible surface (SAS).

In principle, the underlying electrostatic principle of DCSMs is exact. In reality, no well-defined surface exists that separates a solute from a solvent environment, because electron densities of solute and solvent molecules are overlapping. Therefore, cavity definition and molecule-specific cavity construction are crucial steps.

In some embodiments, solvent-accessible surface segments may be constructed in a 3D space. Such embodiments may employ any suitable cavity construction methods known in the art, for example, existing cavity construction methods reviewed by [5].

According to an aspect, a COSMO FINE Cavity construction model [5] may be used, e.g., at step 102 of the method 100 to generate a surface model. A marching tetrahedron algorithm, employed by the FINE model, provides a technique for triangulation to arrive at surface segments. The FINE model further utilizes an atom-type specific COSMO radii based iso-density cavity construction algorithm, which results in a smooth, completely paved cavity of molecular shape. This is of special importance for the COSMO-RS model, which uses the screening charge density on a surface as a main descriptor for a definition of intermolecular interactions. Details of the procedure are given in [5].

### Machine Learning Model(s) for Predicting Charge and Chemical Potential

For each surface segment of a generated surface model, certain embodiments calculate charge (e.g., electric charge) and chemical potential as typical inputs for the COSMO-RS thermodynamic model. In existing methods, charge distributions and chemical potentials are determined via quantum-chemical calculations. In contrast, some embodiments use one or more machine learning models to predict charge and chemical potential of the segments. Compared to existing approaches, certain embodiments thus provide a significant speed-up by several orders of magnitude. Some embodiments reduce calculation time from hours per central processing unit (CPU) core to a few seconds, even at the most accurate level of consideration. Similar to quantum chemistry, the machine learning model-based methods of certain embodiments reflect a special and atomistic environment of a segment in a particular spatial molecular arrangement of neighboring atoms.

Some embodiments may apply one or more machine learning models to predict target properties: segment charge and segment chemical potential. For certain embodiments, quantum-chemical information as input to train the models may be taken or constructed from, e.g., molecular geometries optimized at a density-functional level, such as a B88-VWN-P86 functional and def-TZVP (valence triple-zeta polarization) basis set by, e.g., the TURBOMOLE software and a subsequent single-point calculation at the def2-TZVPD (valence triple-zeta polarization with diffuse functions) basis set, with activated scaled-conductor approximation with infinite dielectric constant ε=∞. Segment charge may include, for example, screening charge or charge density. According to some embodiments, the two quantum-chemical levels may be abbreviated as BP-TZVP and BP-TZVPD-FINE in the nomenclature of the BIOVIA^{®} COSMOtherm^{®} software. Chemical potential in this context is the chemical potential of a segment in response to a change in polarization.

The one or more machine learning models of certain embodiments may be trained against a large amount of COSMO file collections, which serve as a training data set. In an aspect, the training data set may include data for one or more of example molecules, example conformers, example segments, example segment charges, example segment chemical potentials, and example continuum model energies. In an embodiment, the one or more machine learning models may further be validated using known methods and/or using test sets. The COSMO file collections used for training machine learning model(s) of certain embodiments may cover an organic-chemical space including, for example, solvents, industrial chemicals, pharmaceuticals, ionic protonation states, ions, and ionic liquids (cations and anions, also including multiply charged ions). According to some embodiments, a training data set may include conformer sets that contain representatives for both intramolecular hydrogen bonds as well as open (intermolecular) hydrogen bond candidates. In an aspect, a COSMO file collection used for a training data set may include about 16,000 compounds, represented by about 65,000 conformers per quantum-chemical level. According to an implementation, each conformer may on average be built up by ~10³ segments, including particular values for segment areas, charges, and potentials, depending on a size of a molecule and a quantum-chemical level a COSMO file represents. In some embodiments, only a fraction of the available ~10⁸ segments spanning all conformers in the entire dataset are used in a training process. According to one such embodiment, on average, only 3% of segments related to carbon and hydrogen atoms may be used for training and validation (thus, 97% remain for test sets), and only 25% of segments may relate to nitrogen, oxygen, and fluorine atoms (thus, 75% remain for test sets). In an aspect, no threshold is applied for other atom types. According to an embodiment, during training and validation, a fraction of 0.9 of all used segments may be used for training, while a fraction of 0.1 may be set aside for validation.

In an embodiment, because segments in COSMO files already reflect outlying charge correction, no explicit outlying charge correction is necessary. According to an implementation, after processing all segments, a correction of residual charges may be performed to arrive at an overall formal charge. Certain embodiments may also apply corrections to predicted charges using various techniques. Such techniques may include, but are not limited to: (i) alignment of formal charges and overall predicted conformer-based charges and (ii) correction for outlying charges as necessary.

The one or more machine learning models applied by certain embodiments, e.g., at step 102 of the method 100, may include, but are not limited to, artificial neural networks.

In an embodiment, an artificial neural network architecture may include an input layer, one or more hidden layers, which may start with, e.g., a dense hidden layer composed of 256 nodes, and an output layer. Embodiments may employ any neural network architecture known in the art. For example, embodiments may utilize various types of architectures, including, but not limited to, artificial neural networks and deep neural networks. Moreover, neural networks according to embodiments may include additional layers, such as convolutional layers. According to an aspect, neural networks may use a rectified linear unit (ReLU) activation function, an adaptive moment estimation (Adam) solver algorithm, a tolerance of 10⁻⁸, and an early-stopping algorithm. Further, it is noted that embodiments are not limited to a particular number of layers, number of nodes, activation function, algorithm, or tolerance described herein; instead, any suitable number of layers, number of nodes, activation function(s), algorithm(s), or tolerance known in the art may be employed. In an implementation, hyperparameters of the artificial neural network architecture may be subject to further optimization. For example, network parameters may be selected to achieve a desired balance between network size and model performance. In some embodiments, input features may be scaled by removing the mean and scaling to unit variance of a training set. Although neural networks are discussed herein, those of skill in the art will recognize that embodiments are not limited to such techniques; rather, any suitable known machine learning or statistical learning methods may be used.

In some embodiments, the one or more machine learning models may use types of information for input features that are the same as those used by conventional quantum-chemical approaches. Such features may include, for example, atom type, atom-atom distance, atom-segment distance, bond type, bond angle, torsion angle, formal charge, 3D atom position, and various atom-type specific features. According to an embodiment, 3D atom position may be a rotationally invariant representation of atoms' 3D positioning relative to each other. Further, in an implementation, an optional cut-off radius may be applied. Certain embodiments provide a benefit, among others, of making it unnecessary to depend on features such as molecular topology or connectivity, molecular fragments, chemical functional groups, and classic chemo-informatics fingerprints. By avoiding such dependency, some embodiments can determine, e.g., a very generalized chemical description of possible charge distributions. Although certain embodiments are not required to consider these features, they may optionally be used. In an aspect, there is no direct cut-off radius, but a maximum of 24 nearest atoms may be considered as a chemical environment for a particular segment. According to an embodiment, atom types are not encoded directly, but rather indirectly by their quantum-chemical features, e.g., electron affinities, ionization potentials, allowed orbital configurations, atom-type specific radii, etc. This technique increases the applicable domain of the one or more machine learning models according to some embodiments even further. However, in certain embodiments, atom types may be encoded directly. All atom types up to Radon (atomic number 86, i.e., excluding g orbitals), as well as ionic structures, are supported by construction of the one or more machine learning models according to embodiments.

### Machine Learning Model(s) for Predicting Energy

Certain embodiments may further use a supplemental machine learning model (i.e., a machine learning model in addition to the one or more models used at step 102 to predict charge and chemical potential) to predict energy corresponding to a 3D structure model based on the 3D structure model and a generated surface model. As described herein, in an embodiment, the 3D structure model may correspond to a conformer of a molecule.

In some embodiments, the predicted energy may be one or more of: (i) total molecular or ionic energy, (ii) dielectric energy, e.g., dielectric energy in a conductor-like dielectric continuum, and (iii) gas-phase energy. According to an aspect, the same COSMO file collection (discussed hereinabove under the heading "Machine Learning Model(s) for Predicting Charge and Chemical Potential") may be used for training the supplemental machine learning model for each target property and level of calculation (e.g., BP-TZVP and BP-TZVPD-FINE). Likewise, in an implementation, a fraction of 0.9 of each target property may be used for training and a fraction of 0.1 may be set aside for validation. Some embodiments may employ the same artificial neural network architecture and applied hyperparameters as described hereinabove for the supplemental machine learning model. However, in an aspect, a neural network used to predict energy (e.g., total molecular energy, ionic energy, dielectric energy, or gas-phase energy) may include a first hidden layer that is composed of 512 instead of 256 nodes for total and gas-phase energies. Certain embodiments may use similar input features, e.g., atom types, distances, angles, torsions, and formal charge, etc., with the supplemental machine learning model to predict energy. According to an implementation, atom types are not encoded directly, but rather indirectly by their quantum-chemical features, e.g., electron affinities, ionization potentials, allowed orbital configurations, and atom-type specific radii, etc. Some embodiments may predict energies (e.g., conductor or gas-phase state) for atom types such as H (hydrogen), Li (lithium), Be (beryllium), B (boron), C (carbon), N (nitrogen), O (oxygen), F (fluorine), Na (sodium), Mg (magnesium), Si (silicon), P (phosphorus), S (sulfur), K (potassium), Ca (calcium), Cl (chlorine), Se (selenium), Br (bromine), and I (iodine), among other examples.

Although neural networks are discussed here, those of skill in the art will recognize that embodiments are not limited to such techniques; rather, any suitable known machine learning or statistical learning methods may be used by embodiments for predicting energy. Published machine learning models include, for example, the deep-learning architecture for molecules and materials described by [6].

Certain embodiments provide single-point results for pre-defined molecular geometries. Because some embodiments predict energy, for example total molecular energy or ionic energy, such embodiments can also be extended to run geometry optimizations in a condensed environment, by varying the 3D structure model and its optimization towards an energy minimum.

### Writing Output Files

Certain embodiments may collect results, including, e.g., predicted segment charges, segment-wise chemical potentials, and/or energy as described hereinabove, and write the results to an output file, e.g., a dedicated conformer-specific COSMO file storing all COSMO-type information. For instance, the predicted charge and chemical potential determined at step 102 of the method 100 may be written to an output file. For conformer sets, some embodiments may generate multiple COSMO files by running segment construction (described hereinabove under the heading "Construction of Solvent-Accessible Surface Segments") and all predictions (described hereinabove under the headings "Machine Learning Model(s) for Predicting Charge and Chemical Potential" and "Machine Learning Model(s) for Predicting Energy") for each particular conformer separately. Certain embodiments may also generate and store output files, e.g., COSMO files, in the cloud, e.g., the 3DEXPERIENCE^{®} platform. Embodiments are not limited to a particular output file format or cloud platform; instead, any suitable output file format or cloud platform known in the art may be used.

### Application of Output Files in Further Calculations

To demonstrate the application of the novel techniques described herein, output files according to some embodiments, e.g., COSMO files, may be used as input for the BIOVIA^{®} COSMOtherm^{®} software. The software may calculate fluid thermodynamic properties by the COSMO-RS method. Certain embodiments may also, for example, perform calculations via applications provided by the 3DEXPERIENCE^{®} platform (e.g., BIOVIA^{®} Virtual Bench^{®}), or any suitable platform known to those in the art.

Embodiments can successfully predict thermodynamic equilibrium properties, e.g., solubilities, partition coefficients, and/or liquid densities. Further, results show that such properties determined using embodiments closely match known experimentally-determined values. The predictions for multiple chemicals based on conformer sets (e.g., sets of COSMO files) generated by the novel machine learning techniques according to embodiments are in good agreement with predictions generated by conventional quantum-chemical calculations (e.g., density-functional theory). Moreover, the results from machine learning methods according to embodiments outperform those generated by existing approaches.

In addition to thermodynamic property predictions, other examples of further calculations based on output files according to embodiments include, but are not limited to, machine learning models using the generated prediction data as input features (e.g., charge density profiles, energies, and σ-moments, etc.), software applications involving quantum chemistry and/or materials science (e.g., TURBOMOLE and BIOVIA^{®} Materials Studio^{®}), and predictions of biological and/or biochemical properties (e.g., BIOVIA^{®} Discovery Studio^{®}).

### Advantages

Embodiments determine properties of a molecule in an environment, and offer numerous advantages.

For example, the fast machine learning procedures of embodiments reduce the time required to construct and calculate COSMO information for a full conformer set from multiple days or even weeks to a few seconds or even less time.

As another example advantage, embodiments do not require HPC clusters. Embodiments facilitate automating high-throughput screening predictions and the democratization of solutions for thermodynamic equilibrium calculations in the liquid phase. Cloud-based platforms, such as the 3DEXPERIENCE^{®} platform at Dassault Systèmes, benefit from results provided by embodiments that are essentially available instantaneously. In this way, embodiments reduce both cost and carbon footprint; moreover, embodiments increase the platforms' desirability for customers.

Further, as yet another example advantage, embodiments can extend the applicability of COSMO and COSMO-RS to large molecular systems such as biomolecules and polymers, among other examples. Embodiments can also be used to efficiently guide experimentation. Moreover, embodiments provide solubility predictions of polymers that can be leveraged to design sustainable polymers and, thereby, embodiments can improve real-world processes that utilize such polymers, e.g., recycling processes. Because excipients are usually polymer-based, embodiments can additionally enhance pharmaceutical virtual screenings in drug development. In such a setting, efficient methods to screen properties, for example solubility, are needed for focusing experiments and reducing material waste due to inefficient experimental testing. Embodiments provide the needed methods.

Advantageously, embodiments, via the efficient machine-learning procedures, also increase the accuracy of thermodynamic property calculations by avoiding numerical artifacts that arise from derivations of quantum-chemical equations.

### Computer Support

Embodiments can be implemented in existing software and computer-aided design and computer-aided engineering platforms. For example, embodiments can be implemented using features and functionalities of 3DS BIOVIA^{®} software.

FIG. 4 is a simplified block diagram of a computer-based system 440 that may be used to determine properties of a molecule in an environment according to any variety of the embodiments of the present invention described herein. The system 440 comprises a bus 443. The bus 443 serves as an interconnect between the various components of the system 440. Connected to the bus 443 is an input/output device interface 446 for connecting various input and output devices such as a keyboard, mouse, touch screen, display, speakers, etc. to the system 440. A CPU 442 is connected to the bus 443 and provides for the execution of computer instructions. Memory 445 provides volatile storage for data used for carrying out computer instructions. Storage 444 provides non-volatile storage for software instructions, such as an operating system (not shown). The system 440 also comprises a network interface 441 for connecting to any variety of networks known in the art, including wide area networks (WANs) and local area networks (LANs).

It should be understood that the example embodiments described herein may be implemented in many different ways. In some instances, the various methods and machines described herein may each be implemented by a physical, virtual, or hybrid general purpose computer, such as the computer system 440, or a computer network environment such as the computer environment 440, described hereinbelow in relation to FIG. 4. The computer system 440 may be transformed into the machines that execute the methods described herein, for example, by loading software instructions implementing method 100 into either memory 445 or non-volatile storage 444 for execution by the CPU 442. One of ordinary skill in the art should further understand that the system 440 and its various components may be configured to carry out any embodiments or combination of embodiments described herein. Further, the system 440 may implement the various embodiments described herein utilizing any combination of hardware, software, and firmware modules operatively coupled, internally, or externally, to the system 440.

FIG. 5 illustrates a computer network environment 550 in which embodiments of the present invention may be implemented. In the computer network environment 550, the server 551 is linked through the communications network 552 to the clients 553a-n. The environment 550 may be used to allow the clients 553a-n, alone or in combination with the server 551, to execute any of the embodiments described herein. For non-limiting example, computer network environment 550 provides cloud computing embodiments, software as a service (SaaS) embodiments, and the like.

Embodiments or aspects thereof may be implemented in the form of hardware, firmware, or software. If implemented in software, the software may be stored on any non-transient computer readable medium that is configured to enable a processor to load the software or subsets of instructions thereof. The processor then executes the instructions and is configured to operate or cause an apparatus to operate in a manner as described herein.

Further, firmware, software, routines, or instructions may be described herein as performing certain actions and/or functions of the data processors. However, it should be appreciated that such descriptions contained herein are merely for convenience and that such actions in fact result from computing devices, processors, controllers, or other devices executing the firmware, software, routines, instructions, etc.

It should be understood that the flow diagrams, block diagrams, and network diagrams may include more or fewer elements, be arranged differently, or be represented differently. But it further should be understood that certain implementations may dictate the block and network diagrams and the number of block and network diagrams illustrating the execution of the embodiments be implemented in a particular way.

Accordingly, further embodiments may also be implemented in a variety of computer architectures, physical, virtual, cloud computers, and/or some combination thereof, and thus, the data processors described herein are intended for purposes of illustration only and not as a limitation of the embodiments.

The teachings of all patents, published applications, and references cited herein are incorporated by reference in their entirety.

While example embodiments have been particularly shown and described, it will be understood by those skilled in the art that various changes in form and details may be made therein without departing from the scope of the embodiments encompassed by the appended claims.

### References

[1] A. Klamt, G. Schüürmann (1993). "COSMO: a new approach to dielectric screening in solvents with explicit expressions for the screening energy and its gradient". J. Chem. Soc. Perkin Trans. 2 (5): 799-805. doi:10.1039/P29930000799
[2] A. Klamt (2005). "From Quantum Chemistry to Fluid Phase Thermodynamics and Drug Design". Boston, MA, USA: Elsevier. ISBN 9780444519948
[3] P.C. Petris, P. Becherer, J.G.E.M. Fraaije (2021). "Alkane/water partition coefficient calculation based on the modified AM1 method and internal hydrogen bonding sampling using COSMO-RS". J. Chem. Inf. Model. 61 (7): 3453-3462. doi: 10.1021/acs.jcim.0c01478
[4] M. Hornig, A. Klamt (2005). "COSMOfrag: a novel tool for high-throughput ADME property prediction and similarity screening based on quantum chemistry". J. Chem. Inf. Model. 45: 1169-1177. doi:10.1021/ci0501948
[5] A. Klamt, M. Diedenhofen (2018). "A refined cavity construction algorithm for the conductor-like screening model". J. Comput. Chem. 39: 1648-1655. doi: 10.1002/jcc.25342
[6] Schütt et al. (2018). "SchNet - A deep learning architecture for molecules and materials". J. Chem. Phys. 148: 241722 (2018); doi: 10.1063/1.5019779

## Claims

1. A computer-implemented method for determining properties of a molecule in an environment, the method comprising:
constructing one or more three-dimensional (3D) structure models indicating positions of atoms of the molecule; and
determining the properties of the molecule in the environment by, for each 3D structure model of the constructed one or more 3D structure models:
generating a surface model representing the environment, wherein the surface model includes a plurality of segments and the generated surface model defines a relationship between the indicated positions of the atoms of the 3D structure model and the plurality of segments; and
predicting, using a machine learning model, charge and chemical potential of each segment of the plurality of segments based on the 3D structure model and the generated surface model.

2. The method of Claim 1, wherein the machine learning model comprises a first machine learning model and a second machine learning model, and wherein predicting the charge and the chemical potential of each segment of the plurality of segments based on the 3D structure model and the generated surface model comprises:
predicting, using the first machine learning model, electric charge of each segment of the plurality of segments based on the 3D structure model and the generated surface model; and
predicting, using the second machine learning model, the chemical potential of each segment of the plurality of segments based on the 3D structure model and the generated surface model.

3. The method of Claim 1, wherein determining the properties of the molecule in the environment by, for each 3D structure model of the constructed one or more 3D structure models, further comprises:
predicting, using a supplemental machine learning model, energy corresponding to the 3D structure model based on the 3D structure model and the generated surface model, preferably wherein each 3D structure model of the constructed one or more 3D structure models corresponds to a respective conformer of the molecule.

4. The method of Claim 1, wherein the machine learning model comprises a neural network.

5. The method of Claim 4, wherein the neural network comprises one or more hidden layers and the neural network is configured to employ an activation function at one or more nodes of the one or more hidden layers, preferably wherein the activation function is one of a rectified linear activation function and a softmax function.

6. The method of Claim 1, further comprising training the machine learning model based on a training data set.

7. The method of Claim 6, wherein the machine learning model comprises a neural network, and wherein training the machine learning model based on the training data set comprises:
training the neural network by iteratively updating one or more network weights of the neural network based on the training data set, preferably wherein iteratively updating the one or more network weights of the neural network based on the training data set comprises employing one or more of an adaptive moment estimation solver algorithm and an early stopping algorithm.

8. The method of Claim 6, wherein the training data set comprises data for one or more of: example molecules, example conformers, example segments, example segment charges, example segment chemical potentials, and example continuum model energies.

9. The method of Claim 1, wherein predicting, using the machine learning model, the charge and the chemical potential of each segment of the plurality of segments based on the 3D structure model and the generated surface model comprises:
deriving input feature data based on the 3D structure model; and
predicting, using the machine learning model, the charge and the chemical potential of each segment of the plurality of segments based on the 3D structure model, the generated surface model, and the derived input feature data, preferably wherein the derived input feature data comprises an indication of one or more of: atom type, atom-atom distance, atom-segment distance, bond type, bond angle, torsion angle, formal charge, 3D atom position, and atom-type specific features.

10. The method of Claim 1, further comprising:
receiving one or more user requirements;
for each candidate molecule of a plurality of candidate molecules, performing the constructing and the determining the properties; and
selecting a given molecule from among the plurality of candidate molecules based on the determined properties of the given molecule and the received one or more user requirements.

11. The method of Claim 1, wherein predicting, using the machine learning model, the charge and the chemical potential of each segment of the plurality of segments based on the 3D structure model and the generated surface model comprises:
correcting one or more residual charges of the plurality of segments; and
determining an overall formal charge of the plurality of segments based on the corrected one or more residual charges of the plurality of segments, wherein the determined overall formal charge is the predicted charge of the plurality of segments.

12. The method of Claim 1, wherein constructing the one or more 3D structure models indicating the positions of the atoms of the molecule is based on indications of one or more of: atom type, coordinates, and chemical connectivity, preferably wherein constructing the one or more 3D structure models indicating the positions of the atoms of the molecule comprises employing one or more of: rule-based geometrical models, force fields, and quantum-chemically derived geometrical models.

13. The method of Claim 1, wherein generating the surface model representing the environment comprises employing a cavity construction model.

14. A computer program comprising instructions which, when the program is executed by a computer, cause the computer to perform the method of any of Claims 1 to 13.

15. A system comprising a processor coupled to a memory and a graphical user interface, the memory having recorded thereon the computer program of Claim 14.
